(19) Europäisches Patentamt
European Patent Office
Office européen des brevets

(11) EP 1 847 257 B1

(12) EUROPEAN PATENT SPECIFICATION

(45) Date of publication and mention of the grant of the patent:
22.11.2017 Bulletin 2017/47

(51) Int Cl.:
A61K 9/16 (2006.01)          A61K 9/20 (2006.01)
A61K 31/4422 (2006.01)

(21) Application number: 07251609.9

(22) Date of filing: 16.04.2007

(54) **Solid preparation comprising enteric solid dispersion**

Feste Zubereitung eine magensaftresistente Feststoff-Dispersion enthaltend

Préparation solide comprenant une dispersion solide gastrorésistante

(84) Designated Contracting States:
DE FR GB

(30) Priority: 20.04.2006  JP 2006116273
29.03.2007  JP 2007087958

(43) Date of publication of application:
24.10.2007  Bulletin 2007/43

(73) Proprietor: Shin-Etsu Chemical Co., Ltd.
Tokyo 100-0004 (JP)

(72) Inventors:
• Hoshino, Takafumi
c/o Speciality Chem. Res. Center
Joetsu-shi,
Niigata-ken 942-8601 (JP)
• Kusaki, Fumie
c/o Speciality Chem. Res. Center
Joetsu-shi
Niigata-ken 942-8601 (JP)
• Fukui, Ikuo
c/o Speciality Chem. Res. Center
Joetsu-shi,
Niigata-ken 942-8601 (JP)

(74) Representative: HGF Limited
4th Floor
Merchant Exchange
17-19 Whitworth Street West
Manchester M1 5WG (GB)

(56) References cited:
EP-A- 1 133 984          WO-A- 00/00179
WO-A- 02/089773          WO-A- 2005/079748
WO-A2- 03/063831         JP-A- 2004 067 606
US-A1- 2005 158 386

• TAKEUCHI H ET AL: "SPHERICAL SOLID DISPERSION CONTAINING AMORPHOUS TOLBUTAMIDE EMBEDDED IN ENTERIC COATING POLYMERS OR COLLOIDAL SILICA PREPARED BY SPRAY-DRYING TECHNIQUE" CHEMICAL AND PHARMACEUTICAL BULLETIN, PHARMACEUTICAL SOCIETY OF JAPAN, TOKYO, JP, vol. 35, no. 9, 1 January 1987 (1987-01-01), pages 3800-3806, XP001183548 ISSN: 0009-2363

Remarks:
The file contains technical information submitted after the application was filed and not included in this specification

**Description**

BACKGROUND OF THE INVENTION

1. Field of the Invention

The present invention relates to a solid preparation comprising an enteric solid dispersion, the preparation being produced for improving the solubility of a poorly soluble drug. The present invention relates particularly to an enteric solid preparation comprising a solid dispersion, the preparation being able to be rapidly disintegrated and that allows a drug to be dissolved.

2. Description of the Related Art

Poorly soluble drugs have high crystallinity and extremely low solubility in water. Thus, bioavailability or internal absorption of preparations produced from these drugs is low so that there is a problem in that the drug action is insufficient. As a technique for addressing this problem, a solid dispersion has been developed in which molecules of a poorly soluble drug are dispersed in a polymer carrier such as a cellulose derivative, in an amorphous state.

Conventional solid dispersions are used as preparations in the form of capsules containing a solid obtained by spray-drying a cosolvent in which a poorly soluble drug and a carrier are dissolved, or in the form of fine granules or granules as they are. The form of tablets, which is a generally used dosage form of solid preparations, is most preferable because tablets are easily prescribed and administered in a fixed dose, and easily handled and taken by patients.

It is known that in the case of tablets produced from a solid dispersion powder, the porosity of the tablets is often lowered not only due to a reduced specific surface area, but also due to plastic deformation of amorphous drug molecules during a compression-molding process and strong bonding between carrier polymers. This low porosity leads to slow permeation of water molecules into the tablets in administration, and to slow disintegration of the tablets, and thus the solid dispersion cannot exert its original effect of improving the dissolution. Furthermore, the viscosity of a water-soluble or enteric polymer serving as a carrier increases during hydration or dissolution, and thus a type of hydrogel layer is formed on the surface of the tablets during dissolution, so that water is further prevented from infiltrating.

As means for addressing these problems, Japanese Patent Application (PCT National Phase) Unexamined Publication No. 2005-517690 has proposed a tablet comprising a solid dispersion powder, a disintegrator and an excipient comprising porosigen, and obtained by spray-drying,. Furthermore, Japanese Patent Application Unexamined Publication No. 5-262642/1993 has proposed a powder in which a water-soluble polymer base and if necessary, an excipient and a disintegrator are added to a poorly soluble drug. However, an added amount of a concentration-enhancing polymer or a water-soluble polymer base, serving as carriers, is large, and thus the polymer viscosity after administration increases so that the rate at which a drug is dissolved tends to be lowered. Furthermore, in the case of a solid dispersion powder obtained by spray-drying as in Japanese Patent Application (PCT National Phase) Unexamined Publication No. 2005-517690, the particle size of the solid dispersion powder prepared by spray-drying in this manner is small, and thus when it is simply mixed with an excipient, segregation is caused so that ingredients are not distributed uniformly in the powder for tableting. Accordingly, a granulation step is required. More specifically, exemplified is a dry granulation method in which after the solid dispersion powder is mixed with the other ingredients, the mixture is compressed and crushed to form a granulated powder for tableting. Moreover, this process makes the operation complicated, and the solid dispersion may be recrystallized in compression. Furthermore, the disintegrator is added after the solid dispersion has been prepared, and thus when the solid dispersion is aggregated and bonded in the tablet due to high bonding strength of the carrier, aggregation may be formed and dispersed in water during disintegration, lowering the dissolution of the drug.

Furthermore, according to Japanese Patent Application (PCT National Phase) Unexamined Publication No. 2005-517690, a solid dispersion powder is prepared in advance from a poorly soluble drug and a concentration-enhancing polymer, and then a disintegrator and an excipient are physically mixed therewith. Accordingly, the obtained tablet is disintegrated even in the stomach although it is described that a preferable disintegration time is within ten minutes after the tablet is introduced into a disintegration medium. The solid dispersion with a larger specific surface area is exposed for a long time in digestive juice, so that the solubility may be lowered due to recrystallization of the dissolved drug.

Japanese Patent Application Unexamined Publication No. 2004-67606 has proposed a tablet comprising fine granules obtained by spraying a solution containing itraconazole, which is a poorly soluble drug, a water-soluble polymer and an enteric polymer, on a mixed powder of an excipient and a disintegrator, granulating and drying the resultant. However, the amount of the disintegrator added is small and it takes as long as 360 minutes for the drug to be dissolved from the tablet. Thus, the disintegratability of the tablet is not improved.

Hirasawa et al. (Journal of the Pharmaceutical Society of Japan, 124(1), 19-23(2004)) has proposed a tablet produced by loading an ethanol dispersion as a binding liquid containing nilvadipine which is a poorly soluble drug, crospovidone and methylcellulose, into a mixed powder of materials such as lactose, methylcellulose and low-substituted hydroxypropylcellulose, and agitating and granulating the mixture. The nilvadipine is soluble in ethanol, but crospovidone and methylcellulose are not soluble in ethanol. Thus, it seems that the ethanol functions only as an agent for dispersing and diluting amorphous nilvadipine because a co-dissolved state is not obtained. In order to disperse amorphous drug molecules in a polymer serving as a carrier, it is necessary to obtain a co-dissolved state in a cosolvent in which both the drug molecules and the polymer are dissolved. Thus, it seems that the solid dispersion of amorphous nilvadipine described in Journal of the Pharmaceutical Society of Japan 124(1), 19-23(2004) does not have sufficient solubility. Furthermore, since an amount of water-soluble polymer added is large, it may be difficult to obtain a preparation that can be rapidly dissolved. WO 03/063831 relates to immediate release dosage forms containing solid drug dispersions.

## SUMMARY OF THE INVENTION

The present invention was reached in view of the above-described circumstances and provides a solid preparation comprising an enteric solid dispersion that allows a drug in the preparation to be rapidly dissolved without impairing the dissolution of the solid dispersion, and a method for producing the same.

The inventors had conducted an in-depth study in order to address the above-described problem, and found that a solid preparation produced by partly or entirely covering a mixed powder comprising at least an excipient and a disintegrator with a solid dispersion comprising a poorly dissoluble drug and an enteric polymer, has excellent dissolution in forms of granules, tablets or the like without lowering disintegration of tablets obtained by compression-molding. Thus, the present invention has been achieved.

More specifically, the present invention provides a solid preparation comprising a poorly soluble drug, an enteric polymer, an excipient and a disintegrator as specified in appended claim 1. In one of the preferable embodiments, the content of the enteric polymer may be 1 to 37% by weight and the content of the disintegrator may be 15 to 50% by weight. The present invention further provides a method for producing a solid preparation as specified in appended claim 4.

According to the present invention, obtained is a solid preparation with excellent solubility which can have high solubility in the form of a granulated product, and which can be disintegrated and can release at least 70% by weight of a poorly soluble drug within 10 minutes after the introduction into an appropriate dissolution medium in the form of a tablet.

## DETAILED DESCRIPTION OF THE INVENTION

Hereinafter, the present invention is described in more detail.

A poorly soluble drug used in the present invention has extremely low solubility in water, and poor absorption in ordinary oral administration. For example, the poorly soluble drug means a drug that is "practically insoluble or insoluble" or "very slightly soluble" as prescribed in the Japanese Pharmacopoeia Fourteenth Edition. The term "solubility" of a drug in the Japanese Pharmacopoeia Fourteenth Edition means the degree of dissolution of the drug, powdered in the case of a solid, within 30 minutes in a solvent at 20 $\pm$ 5°C, by shaking for 30 seconds each time at 5-minute intervals. If a drug is "practically insoluble or insoluble", then an amount of solvent (water, in this specification) required for dissolving 1 g or 1 ml of the drug is 10,000 ml or more. If a drug is "very slightly soluble", then an amount of solvent required for dissolving 1 g or 1 ml of the drug is 1,000 ml or more and less than 10,000 ml.

Specific examples of the poorly soluble drug used in the present invention may include, but are not limited to, nifedipine, phenacetin, phenytoin, digitoxin, nilvadipine, diazepam, griseofulvin and chloramphenicol.

An enteric polymer is used as a carrier of a solid dispersion of the present invention. Use of an enteric polymer as a carrier of a solid dispersion is advantageous due to the dissolving properties of an enteric polymer in which release of a drug from the solid preparation is suppressed in the stomach, and the drug is completely released and dissolved out of the solid dispersion after the solid dispersion has moved from the stomach to the small intestine. In other words, the drug of the solid dispersion can be specifically and efficiently dissolved and absorbed in the small intestine having the largest absorption area and the highest absorbability of drugs in the body. This is effective also for a drug having a possibility of recrystallization after dissolution, which is regarded as a general problem of solid dispersion preparations. More specifically, when an enteric polymer is used as a carrier of a solid dispersion for a drug that may be recrystallized during a period in which the preparation moves from the stomach to the intestines, recrystallization is suppressed and thus the solubility of the drug does not return to its original low solubility due to recrystallization. Consequently, the drug in the solid dispersion can be specifically and efficiently dissolved and absorbed in the small intestine.

According to the condition prescribed in the Japanese Pharmacopoeia Fourteenth Edition, an enteric polymer belongs to a polymer that is "practically insoluble or insoluble (the amount of water required for dissolving 1 g or 1 ml of the drug is 10,000 ml or more)", and can be dissolved in an alkaline solution. Specific examples of the enteric polymer may include cellulose acetate phthalate, cellulose acetate trimellitate, cellulose acetate succinate, methylcellulose phthalate, hydroxymethylcellulose ethyl phthalate, hydroxypropylmethylcellulose phthalate, hydroxypropylmethylcellulose acetate succinate, hydroxypropylmethyl acetate maleate, hydroxypropylmethyl trimellitate, carboxymethylethylcellulose, polyvinyl butyrate phthalate, polyvinyl alcohol acetate phthalate, methacrylic acid/ethyl acrylate copolymer (preferably in a weight ratio of 1:99 to 99:1), and methacrylic acid/methyl methacrylate copolymer (preferably in a weight ratio of 1:99 to 99:1). Hydroxypropylmethylcellulose phthalate, hydroxypropylmethylcellulose acetate succinate, hydroxypropylmethyl acetate maleate and hydroxypropylmethyl trimellitate may be preferable and hydroxypropylmethylcellulose acetate succinate may be particularly preferable.

Hydroxypropylmethylcellulose acetate succinate which can start dissolution of a drug rapidly after it shifts from the stomach to an upper or middle part of the small intestine may be preferable. More specifically, hydroxypropylmethylcellulose acetate succinate which can be dissolved within 120 minutes in the Japanese Pharmacopoeia phosphate buffer liquid having a pH value of 5 to 7 (5.0 to 6.8) may be preferable. A factor which controls a solubility of hydroxypropylmethylcellulose acetate succinate polymer may be a content of each substituent and a composition ratio of to acetyl to succinoyl groups. The hydroxypropylmethylcellulose acetate succinate may include, but not limited to, the following examples:

Specific Example 1: 20 to 24% by weight methoxyl groups, 5 to 9% by weight hydroxypropoxyl groups, 5 to 9% by weight acetyl groups, 14 to 18% by weight succinoyl groups, and the composition ratio of 1.5 to 2.

Specific Example 2: 21 to 25% by weight methoxyl groups, 5 to 9% by weight hydroxypropoxyl groups, 7 to 11% by weight acetyl groups, 10 to 14% by weight succinoyl groups, and the composition ratio of 0.9 to 2.0

Hydroxypropylmethylcellulose acetate succinate not having the above contents of substituents can be used as long as it can be dissolved within 120 minutes in the Japanese Pharmacopoeia phosphate buffer liquid having a pH value of 5 to 7 (5.0 to 6.8) by combining it with Specific Example 1 and/or Specific Example 2 of hydroxypropylmethylcellulose acetate succinate.

The content of the enteric polymer may be preferably 1 to 37% by weight with respect to the total amount of the solid preparation. If the content of the enteric polymer is less than 1% by weight, then it may be difficult to obtain a completely amorphous state of the poorly soluble drug in the solid dispersion. If the content is more than 37% by weight, then the ratio of the enteric polymer in the preparation becomes large, which is not preferable because the dose may become large in the form of granulated products and the size of the preparation become large in the form of tablets.

The weight ratio of the poorly soluble drug to the enteric polymer may be preferably 1 : (1 to 5). If the ratio of the enteric polymer is less than 1, then the poorly soluble drug in the solid dispersion may not be in a completely amorphous state. If the ratio is more than 5, then the ratio of the enteric polymer in the preparation becomes large, and thus the size of the preparation becomes large, which may not be suitable for a generally used preparation.

The solvent for preparing the solid dispersion comprising the enteric polymer and the poorly soluble drug may be preferably a solvent in which the poorly soluble drug is well dissolved and the enteric polymer is also dissolved. Examples of the solvent may include methanol, ethanol, methylene chloride, acetone, a mixture thereof and their mixed solvents with water. The solvent may be selected appropriately based on the solubility of the poorly soluble drug and the enteric polymer in the solvent.

The solvent may be added in an amount at which the solid concentration is preferably 3 to 18% by weight, particularly preferably 3.5 to 12% by weight.

If necessary, a surfactant such as polyethylene glycol, polyethylene oxide and polypropylene glycol may be added to the solid dispersion as a third ingredient.

Examples of the excipient used in the present invention may include lactose, cornstarch, saccharose, mannite, anhydrous calcium phosphate, crystalline cellulose and their mixtures. It may be particularly preferable to use a mixed powder comprising lactose and cornstarch in a weight ratio of 7:3.

The content of the excipient may be preferably 2 to 90% by weight, particularly preferably 5 to 60% by weight, with respect to the total amount of the preparation. If the content of the excipient is less than 2% by weight, then the amount of the disintegrator may become too large and thus the flowability of the granulated powder may be poor. If the content is more than 90% by weight, then the amount of the disintegrator becomes small, and thus an effect of improving solubility may not be expected.

The disintegrator used in the present invention is low-substituted hydroxypropylcellulose because it provides granulates with high flowability and ensures high dissolution from the compression-molded preparation when it has a loose bulk density of 0.40 g/ml or more and a tapped bulk density of 0.60 g/ml or more.

Herein, the term "loose bulk density" means the bulk density in a loosely filled state and is measured by uniformly supplying a sample from 23 cm above, through a sieve with 24 mesh of Japanese Industrial Standards (JIS), to a cylindrical vessel of stainless steel having a diameter of 5.03 cm and a height 5.03 cm (volume 100 ml), and performing weighing after leveling at the upper surface. The term "tapped bulk density" means the bulk density in a tightly filled state obtained by performing tapping on the vessel in the loosely filled state. The tapping means an operation to make the sample be tightly filled, by repeatedly dropping the vessel filled with the sample from a predetermined height thereby providing the bottom portion with a light impact. In practice, when weighing has been completed after leveling at the upper surface in the measurement of the loose bulk density, a cap (a component of a powder tester manufactured by Hosokawa Micron Corporation) is placed on the vessel, powder is added to the upper edge of the cap, and tapping is performed 180 times at a tapping height of 1.8 cm. When the tapping has been completed, the cap is removed and weighing is performed after leveling the powder at the upper surface of the vessel. The bulk density in this state is taken as the tapped bulk density. These operations can be performed using a powder tester (PT-D) manufactured by Hosokawa Micron Corporation.

The low-substituted hydroxypropylcellulose in the present invention has a degree of compression of 35% or less. Herein, the degree of compression means the degree of volume decreased and is obtained by the following equation:

$$\text{Degree of compression (\%) =}$$

$$\{(\text{tapped b.d.} - \text{loose b.d.}) / \text{tapped b.d.}\} \times 100$$

wherein b.d. means bulk density.

The content of the disintegrator may be preferably 15 to 50% by weight, particularly preferably 20 to 40% by weight, with respect to the total amount of the preparation. If the content of the disintegrator is less than 15% by weight, then an effect of improving solubility may be weak and an expected effect may not be obtained. If the content is more than 50% by weight, then the flowability of the obtained granule powder may be lowered, which is not preferable for powder for tableting.

If necessary, a lubricant may be added to the tablet of the solid dispersion of the present invention. Examples of the lubricant may include magnesium stearate, sucrose fatty acid ester, polyethylene glycol, talc and stearic acid.

The amount of the lubricant may be preferably 0.5 to 2% by weight with respect to the amount of the preparation excluding the lubricant. If the amount of the lubricant added is less than 0.5% by weight, then sufficient lubricative properties may not be obtained so that the preparation may adhere to a mortar or a pestle during tableting. If the amount is more than 2% by weight, then hardness or disintegratability may be lowered.

The tablets obtained in the present invention can restrain disintegration of the tablets and dissolution of the drug in an acidic environment in the stomach, while they are disintegrated in a neutral to alkaline environment in the intestine. Thus, the solubility of the drug is improved. The reason for this is that when the granulated product as powder for tableting are prepared, a mixed powder comprising an excipient and an disintegrant is partly or entirely covered with a solid dispersion comprising a poorly soluble drug and an enteric polymer so that the enteric polymer serving as a carrier is attached to the surface of the disintegrator. In the tablets using the granulated product, disintegration of the tablets is suppressed in the stomach because water hardly permeates into the internal portion of the tablets, while disintegration of the tablets takes place in the intestines in which the carrier starts to be dissolved, enabling water to permeate and the disintegrator to swell. As a result, the surface area of particles of the solid dispersion is increased so that the solubility is increased.

Next, methods for producing granulated products and tablets as typical dosage forms of the solid preparation of the present invention are described.

The granulated product of the solid preparation of the present invention can be obtained by spraying an enteric polymer solution in which the poorly soluble drug has been dispersed or dissolved, on a mixed powder comprising the excipient and the disintegrator, and granulating and drying the resultant. More specifically, the enteric polymer solution in which the poorly soluble drug has been dispersed or dissolved is sprayed on the mixed powder of the excipient and the disintegrator which is flowing in a granulator, the resultant is granulated and dried, and then the particle regulation is carried out.

Examples of the granulator may include a fluidized bed granulation coating device, a high speed agitation granulating device and a rolling granulating device. The fluidized bed granulation coating device may be particularly preferable.

The granulated product thus obtained may include powders and granules. The granulated product may be used as a filler in a capsule.

Using the granulated product obtained by the above-described method as powder for tableting, the tablets of the present invention may be obtained by adding an optional lubricant to the granulated product and compression-molding the granulated product in a tableting machine. The granulated product may be optionally pulverized before the tableting using an appropriate pulverizer in view of improving powder properties or dissolution abilities. The pulverizer may include a knife mill, a roller mill, a ball mill, a jet mill, a screen mill and a beads mill.

When the thus obtained granulated product of the solid preparation is evaluated using the Japanese Pharmacopoeia 2nd fluid (artificial intestinal juice) having a pH value of 6.8 in accordance with "Dissolution Test" in the Japanese Pharmacopoeia Fourteenth Edition, the dissolution of the drug within 5 minutes after administration may be 70% or more with respect to the initial concentration of the drug administered. Thus, high dissolution is exhibited. Furthermore, when the "Dissolution Test" using the Japanese Pharmacopoeia 1st fluid (artificial gastric juice) having a pH value of 1.2 is carried out, the dissolution of the drug after 120 minutes elapse may be 10% by weight or less of the initial concentration of the drug administered. Thus, the granulated product is not disintegrated in the stomach.

On the other hand, when the obtained tablets of the solid preparation are evaluated using the Japanese Pharmacopoeia 2nd fluid (artificial intestinal juice) having a pH value of 6.8 in accordance with "Disintegration Test" in the Japanese Pharmacopoeia Fourteenth Edition, the tablets can be disintegrated within 10 minutes after the administration. The tablets may have disintegration time of 20 minutes or higher in the Japanese Pharmacopoeia 1st fluid (artificial gastric juice) having a pH value of 1.2. Thus, high disintegration property is exhibited.

Moreover, when the obtained tablets of the solid preparation are evaluated using the Japanese Pharmacopoeia 2nd fluid (artificial intestinal juice) having a pH value of 6.8 in accordance with "Dissolution Test" in the Japanese Pharmacopoeia Fourteenth Edition, the dissolution within 10 minutes after the administration can be 70% or more with respect to the initial concentration of the drug administered. Thus, high dissolution is exhibited. When the obtained tablets of the solid preparation are evaluated using the Japanese Pharmacopoeia 1sd fluid (artificial gastric juice) having a pH value of 1.2 in accordance with "Dissolution Test", the dissolution after 120 minutes elapse can be 10% or less with respect to the initial concentration of the drug administered. Thus, no dissolution takes place in the stomach.

The solid preparation obtained in the present invention may be coated by known methods in order to provide taste-masking or odor-masking, to make the preparation enteric, or to achieve sustained release of the preparation. Examples of the coating agent may include water-soluble polymers, for example, alkylcellulose such as methylcellulose, hydroxyalkylcellulose such as hydroxyethylcellulose and hydroxypropylcellulose, hydroxyalkylalkylcellulose such as hydroxyethylmethylcellulose and hydroxypropylmethylcellulose, polyvinyl alcohol, polyvinyl pyrrolidone; enteric polymers such as cellulose acetate phthalate, methacrylic acid copolymer L, methacrylic acid copolymer LD, methacrylic acid copolymer S, hydroxypropylmethylcellulose phthalate, hydroxypropylmethylcellulose acetate succinate and carboxymethylethylcellulose; and polymers soluble in stomach such as polyvinyl acetal diethylaminoacetate and aminoalkyl methacrylate copolymer.

Hereinafter, the present invention is specifically described by way of examples and comparative examples, but it should not be construed that the present invention is limited to these examples.

Example 1 and Comparative Example 1

The weight of each component was chosen so that the total solid contents were 240g as shown in Table 1. A solid dispersion solution was prepared by dissolving nifedipine and a various enteric polymer in amounts described in Table 1, in a mixed solvent containing ethanol and water in a weight ratio of 8:2. The solid dispersion solution was sprayed on a mixture of low-substituted hydroxypropylcellulose (L-HPC) (10.9% by weight of hydroxypropoxyl groups), lactose (Pharmatose manufactured by DMV International Co., Ltd.) and cornstarch (cornstarch W manufactured by Nihon Shokuhin Kako Co., Ltd.) which had been flowing in a fluidized bed granulation coating device (Multiplex MP-01 manufactured by POWREX CORPORATION), and the resultant was granulated and dried. Then the particle size was regulated with a sieve of 30 mesh (opening: 500 $\mu$m) to yield granulated product. As a comparative example, a granulated product containing no low-substituted hydroxypropylcellulose serving as a disintegrator and granules comprising the solid dispersion containing hydroxypropylcellulose acetate succinate having different pH-dependent solubility wherein it is soluble at pH of 6.8 or higher were produced in same manners.

The results of the flowability evaluation are shown in Table 1 wherein 20 g each of the obtained granulated products having Formulae A to K was evaluated using the orifice flowability index. Herein, the orifice flowability index means the index for evaluating the flowability of powder and is obtained by placing 20 g of sample in an hourglass-shaped funnel (inner diameter: 42 mm, height: 90 mm) with its orifice blocked, allowing the sample to flow down through the orifice, and evaluating the flowability based on the orifice size.

The evaluation of the flowability is classified into "good" which means flowability is particularly excellent so that the powder flows down rapidly and "poor" which means flowability is poor so that the powder does not flow down through the orifice.

The granulated products having Formulae A to K in Example 1 belonged to "good" and had superior flowability to those having Formulae L and M in Comparative Example 1 which belonged to "poor".

Table 1

| | | composition | | | | | | total weight (g) | flowability |
|---|---|---|---|---|---|---|---|---|---|
| | | nifedipine (g) | HPMCAS (g) | HPMCP (g) | L-HPC (g) | lactose (g) | cornstarch (g) | | |
| Example 1 | Formula A | 6 | 12 *1 | | 48 | 121.8 | 52.2 | 240 | good |
| | Formula B | 6 | 12 *1 | | 96 | 88.2 | 37.8 | 240 | good |
| | Formula C | 12 | 24 *1 | | 24 | 126 | 54 | 240 | good |
| | Formula D | 12 | 24 *1 | | 48 | 109.2 | 46.8 | 240 | good |
| | Formula E | 12 | 24 *1 | | 96 | 75.6 | 32.4 | 240 | good |
| | Formula F | 18 | 36 *1 | | 96 | 72 | 18 | 240 | good |
| | Formula G | 43.2 | 86.4 *1 | | 96 | 11.52 | 2.88 | 240 | good |
| | Formula H | 12 | 24 *2 | | 96 | 75.6 | 32.4 | 240 | good |
| | Formula I | 12 | | 24 *3 | 96 | 15.6 | 32.4 | 240 | good |
| | Formula J | 18 | | 36 *3 | 96 | 72 | 18 | 240 | good |
| | Formula K | 43.2 | | 86.4 *3 | 96 | 11.52 | 2.88 | 240 | good |
| Comp. Ex.1 1 | Formula L | 12 | 24 *1 | | - | 163.2 | 40.8 | 240 | poor |
| | Formula M | 12 | | 24 *3 | - | 163.2 | 40.8 | 240 | poor |

*1 hydroxypropylmethylcellulose acetate succinate (23.0wt% methoxyl groups, 7.4wt% hydroxypropoxyl groups, 9.3wt% acetyl groups and 11. 0wt% succinoyl groups)
*2 hydroxypropylmethylcellulose acetate succinate (23.5wt% methoxyl groups, 7.4wt% hydroxypropoxyl groups, 7.3wt% acetyl groups and 14. 8wt% succinoyl groups)
*3 hydroxypropylmethylcellulose phthalate (19.2wt% methoxyl groups, 6.1wt% hydroxypropoxyl groups and 33.2wt% carboxybenzoyl groups)

Example 2 and Comparative Example 2

The granulated products obtained in Example 1 and Comparative Example 1 were weigh into 3780mg for each of Formulae A and B, 1890mg for each of Formulae C to E, H, I, L and M, 1260mg for each of Formulae F and J, and 525mg for each of Formulae G and K so that the content of nifedipine drug was 94.5mg. They were tested in accordance with the Paddle Method of the Dissolution Test in the Japanese Pharmacopoeia Fourteenth Edition. For the sake of reference, 94.5 mg of nifedipine powder alone was also tested in the same manner. As for the conditions for the Dissolution Test, the rotational speed was 100 rpm, and 900 ml of the Japanese Pharmacopoeia 2nd fluid (artificial intestinal juice, pH 6.8) was used as a test fluid. The results are shown in Table 2.

When each of the granulated products (Formulae A to K) of Example 2 was evaluated using the Japanese Pharmacopoeia 1sd fluid (artificial gastric juice) having a pH value of 1.2 in accordance with "Dissolution Test" in the Japanese Pharmacopoeia Fourteenth Edition, the dissolution after 120 minutes elapse was 10% or less with respect to the initial concentration of the drug administered. When it was evaluated using the Japanese Pharmacopoeia 2nd fluid (artificial intestinal juice) having a pH value of 6.8 in accordance with "Dissolution Test" in the Japanese Pharmacopoeia Fourteenth Edition, the dissolution within 10 minutes was 70% or more. Accordingly, the granulated products in Example 2 exhibited higher dissolution of the drug than the granulated products having Formulae L and M in Comparative Example 2. In the dissolution test using the Japanese Pharmacopoeia 2nd fluid, the granulated products in Example 2 exhibited significantly high dissolution concentration of the drug and dissolution in comparison with the solubility of nifedipine powder alone.

As a result, it is evident that the granulated products of the solid preparation of the present invention have excellent dissolution.

Table 2

| dissolution test | | dissolution of drug(%) | | | | | | | | | |
|---|---|---|---|---|---|---|---|---|---|---|---|
| | | in the Japanese Pharmacopoeia (JP) 2nd Fluid | | | | | | | | | in the JP 1st Fluid |
| test time (minutes) | | 0 | 2 | 5 | 10 | 15 | 20 | 30 | 45 | 60 | 120 |
| Example 2 | Formula A | 0 | 100.0 | 100.0 | 98.9 | 90.4 | 82.0 | 72.1 | 62.2 | 57.9 | 10% or less |
| | Formula B | 0 | 100.0 | 100.0 | 100.0 | 90.1 | 81.7 | 69.0 | 62.0 | 57.7 | 10% or less |
| | Formula C | 0 | 100.0 | 100.0 | 100.0 | 100.0 | 100.0 | 94.4 | 83.1 | 76.1 | 10% or less |
| | Formula D | 0 | 100.0 | 100.0 | 100.0 | 100.0 | 100.0 | 91.5 | 77.4 | 69.0 | 10% or less |
| | Formula E | 0 | 100.0 | 100.0 | 100.0 | 100.0 | 86.9 | 86.1 | 71.1 | 65.5 | 10% or less |
| | Formula F | 0 | 100.0 | 100.0 | 100.0 | 100.0 | 96.0 | 84.0 | 73.0 | 65.0 | 10% or less |
| | Formula G | 0 | 46.0 | 75.0 | 89.0 | 90.0 | 90.0 | 89.0 | 87.0 | 86.0 | 10% or less |
| | Formula H | 0 | 72.4 | 70.8 | 54.7 | 41.4 | 37.3 | 34.7 | 32.3 | 31.5 | 10% or less |
| | Formula I | 0 | 73.3 | 70.3 | 60.0 | 27.0 | 27.0 | 23.0 | 21.0 | 21.0 | 10% or less |
| | Formula J | 0 | 81.3 | 74.3 | 54.0 | 31. 0 | 28.0 | 28.0 | 27.0 | 24.0 | 10% or less |
| | Formula K | 0 | 88.5 | 71.4 | 54.0 | 33.0 | 30.0 | 30.0 | 29.0 | 24.0 | 10% or less |
| Comp. Ex.2 | formula L | 0 | 47.2 | 57.1 | 61.3 | 59.6 | 58.8 | 57.1 | 54.6 | 55.5 | 10% or less |
| | formula M | 0 | 46.5 | 41.4 | 31.1 | 28.1 | 28.1 | 22.2 | 22.2 | 22.2 | 10% or less |
| nifedipine alone | | 0 | 0.6 | 3.4 | 9.3 | 10.0 | 10.6 | 11.9 | 11.9 | 11.9 | |

Example 3 and Comparative Example 3

The granulated products prepared using Formulae A to K in Example 1 were used as powder for tableting, magnesium stearate as a lubricant was added thereto in the amount of 0.5% by weight relatively to the amount of the powder for tableting and mixed. The resulting mixture was treated in a rotary tableting machine (Vergo manufactured by Kikusui Seisakusho Ltd.) to produce 210 mg of tablets (Formulae a to k). As a comparative example, the granulated product prepared using Formula L and M in Comparative Example 1 were used as powder for tableting and tablets (Formulae 1 and m) were produced in the same manner as in Example 3. The obtained tablets were tested in terms of hardness and disintegratability. The results are shown in Table 3.

The tablets (Formulae a to k) obtained in Example 3 exhibited appropriate hardness and excellent disintegratability in the Japanese Pharmacopoeia 2nd fluid.

On the other hand, although the tablets (Formulae 1 and m) in Comparative Example 3 containing no low-substituted hydroxypropylcellulose as the disintegrator, exhibited good hardness and good disintegratability in the Japanese Pharmacopoeia 2nd fluid, they had disintegration time of 20 minutes or less in the Japanese Pharmacopoeia 1st fluid (pH 1.2).

Table 3

| | | composition | | | | | | total weight (g) | hardness of tablet (kgf) | disintegration (minutes) | |
|---|---|---|---|---|---|---|---|---|---|---|---|
| | | nifedipine (g) | HPMCAS (g) | HPMCP (g) | L-HPC (g) | lactose (g) | cornstarch (g) | | | in JP 1st fluid | in JP 2nd fluid |
| Example 3 | Formula a | 6 | 12 *1 | | 48 | 121.8 | 52.2 | 240 | 5.5 | 25.6 | 3.5 |
| | Formula b | 6 | 12 *1 | | 96 | 88.2 | 37.8 | 240 | 6.2 | 24.4 | 2 |
| | Formula c | 12 | 24 *1 | | 24 | 126 | 54 | 240 | 9.7 | >120 | 9.4 |
| | Formula d | 12 | 24 *1 | | 48 | 109.2 | 46.8 | 240 | 8.9 | >120 | 8 |
| | Formula e | 12 | 24 *1 | | 96 | 75.6 | 32.4 | 240 | 9.6 | >120 | 7.1 |
| | Formula f | 18 | 36 *1 | | 96 | 72 | 18 | 240 | 7.8 | >120 | 1.3 |
| | Formula g | 43.2 | 86.4 *1 | | 96 | 11.52 | 2.88 | 240 | 7.8 | >120 | 4.9 |
| | Formula h | 12 | 24 *2 | | 96 | 75.6 | 32.4 | 240 | 8.9 | >120 | 4.2 |
| | Formula i | 12 | | 24 *3 | 96 | 75.6 | 32.4 | 240 | 6.6 | 29 | 3.6 |
| | Formula j | 18 | | 36 *3 | 96 | 72 | 18 | 240 | 7.9 | >120 | 4.2 |
| | Formula k | 43.2 | | 86.4 *3 | 96 | 11.52 | 2. 88 | 240 | 7.6 | >120 | 4.3 |
| Comp. Ex. 3 | Formula l | 12 | 24 *1 | | - | 163.2 | 40.8 | 240 | 11.3 | 17.5 | 5.3 |
| | Formula m | 12 | | 24 *3 | - | 163.2 | 40.8 | 240 | 10 | 10.6 | 4.3 |

*1 hydroxypropylmethylcellulose acetate succinate (23.0wt% methoxyl groups, 7.4wt% hydroxypropoxyl groups, 9.3wt% acetyl groups and 11.0wt% succinoyl groups)
*2 hydroxypropylmethylcellulose acetate succinate (23.5wt% methoxyl groups, 7.4wt% hydroxypropoxyl groups, 7.3wt% acetyl groups and 14.8wt% succinoyl groups)
*3 hydroxypropylmethylcellulose phthalate (19.2wt% methoxyl groups, 6.1wt% hydroxypropoxyl groups and 33.2wt% carboxybenzoyl groups)

Example 4 and Comparative Example 4

The tablets obtained in Example 3 and Comparative Example 3 were weigh into 1890mg for each of Formulae a and b, 945mg for each of Formulae c to e, h, i, 1 and m, 630mg for each of Formulae f and j, and 265mg for each of Formulae g and k so that the content of nifedipine drug was 47.25mg. They were subjected to the dissolution test in the same manner as in Example 2. For the sake of reference, 47.25 mg of nifedipine powder alone was also tested in the same manner. The results are shown in Table 4.

When each of the tables (Formulae a to k) in Example 3 was evaluated using the Japanese Pharmacopoeia 1sd fluid, the dissolution of the drug after 120 minutes elapse was 10% or less with respect to the initial concentration of the drug administered. When it was evaluated using the Japanese Pharmacopoeia 2nd fluid, the dissolution within 10 minutes was 70% or more. Thus, they exhibited excellent dissolution of the drug. In the dissolution test using the Japanese Pharmacopoeia 2nd fluid, the tablets exhibited significantly high dissolution concentration of the drug and dissolution in comparison with the solubility of nifedipine powder alone.

On the other hand, the tablets for Formulae 1 and m containing no low-substituted hydroxypropylcellulose as the disintegrator, using the Japanese Pharmacopoeia 1sd fluid (artificial gastric juice), the dissolution of the drug after 120 minutes elapse was 10% or less with respect to the initial concentration of the drug administered. However, using the Japanese Pharmacopoeia 2nd fluid, the dissolution within 10 minutes did not reach 70% or more. Thus, the dissolution was not enhanced.

As a result, it is evident that the tablets of the solid preparation of the present invention have excellent disintegration and dissolution.

Table 4

| dissoution test | | dissolution of drug (%) in the Japanese Pharmacopoeia (JP) 2nd Fluid | | | | | | | | | in the JP 1st Fluid |
|---|---|---|---|---|---|---|---|---|---|---|---|
| test time (minutes) | | 0 | 2 | 5 | 10 | 15 | 20 | 30 | 45 | 60 | 120 |
| Example 4 | Formula a | 0 | 56. 1 | 71.7 | 78. 1 | 87.4 | 87.4 | 87.4 | 84.2 | 84.2 | 10% or less |
| | Formula b | 0 | 78.5 | 97.3 | 100. 0 | 100. 0 | 97.3 | 97.3 | 97.3 | 97.3 | 10% or less |
| | Formula c | 0 | 54.0 | 73.5 | 73.5 | 69.8 | 60.5 | 50.6 | 45. 7 | 45.0 | 10% or less |
| | Formula d | 0 | 54.5 | 87.4 | 80.0 | 70.2 | 60.3 | 54. 2 | 48.0 | 48.0 | 10% or less |
| | Formula e | 0 | 70.6 | 85.4 | 62.5 | 57.4 | 53.0 | 49.3 | 45.6 | 45.6 | 10% or less |
| | Formula f | 0 | 59.2 | 66.7 | 71.4 | 71.4 | 67.9 | 60.5 | 50.6 | 45.7 | 10% or less |
| | Formula g | 0 | 34.9 | 61.0 | 75.0 | 90.7 | 100.0 | 99.4 | 94.2 | 94.2 | 10% or less |
| | Formula h | 0 | 72.4 | 70.8 | 54.7 | 41.4 | 37.3 | 34.7 | 32.3 | 31.5 | 10% or less |
| | Formula i | 0 | 54.0 | 66.5 | 71.0 | 72.8 | 72.8 | 69.7 | 60.1 | 60.1 | 10% or less |
| | Formula j | 0 | 51.9 | 67.1 | 71.8 | 71.4 | 62.7 | 54.0 | 47.6 | 43.2 | 10% or less |
| | Formula k | 0 | 48.0 | 69.8 | 72.4 | 72.0 | 67.9 | 60.5 | 50.6 | 45.7 | 10% or less |
| Comp. Ex. 4 | formula l | 0 | 6.6 | 14.9 | 19.9 | 28.2 | 35.6 | 41.4 | 41.2 | 50.5 | 10% or less |
| | formula m | 0 | 37.6 | 40. 4 | 34.9 | 30.7 | 27.9 | 23.7 | 19.5 | 18.1 | 10% or less |
| nifedipine alone | | 0 | 0.6 | 3. 4 | 9.3 | 10. 0 | 10.6 | 11.9 | 11.9 | 11.9 | |

**Claims**

1. A solid preparation comprising a poorly soluble drug, an enteric polymer, an excipient and a disintegrator, wherein a mixed powder comprising at least the excipient and the disintegrator is partly or entirely covered with a solid dispersion comprising the poorly soluble drug and the enteric polymer, wherein said disintegrator is low-substituted hydroxypropylcellulose having 5 to 16% by weight of hydroxypropoxyl groups, a loose bulk density of 0.40 g/ml or higher and a tapped bulk density of 0.60 g/ml or higher, and wherein the low-substituted hydroxypropylcellulose has a degree of compression of 35% or less, wherein degree of compression (%) is [(tapped bulk density-loose bulk density)/tapped bulk density] x 100.

2. The solid preparation according to claim 1, wherein a content of said enteric polymer is 1 to 37% by weight and a content of said disintegrator is 15 to 50% by weight.

3. The solid preparation according to claim 1 or 2, wherein said enteric polymer is selected from the group consisting of cellulose acetate phthalate, cellulose acetate trimellitate, cellulose acetate succinate, methylcellulose phthalate, hydroxymethylcellulose ethyl phthalate, hydroxypropylmethylcellulose phthalate, hydroxypropylmethylcellulose acetate succinate, hydroxypropylmethyl acetate maleate, hydroxypropylmethyl trimellitate, carboxymethylethylcellulose, polyvinyl butyrate phthalate, polyvinyl alcohol acetate phthalate, methacrylic acid/ethyl acrylate copolymer, and methacrylic acid/methyl methacrylate copolymer.

4. A method for producing a solid preparation, comprising the steps of spraying an enteric polymer solution, in which a poorly soluble drug has been dispersed or dissolved, on a mixed powder comprising an excipient and a disintegrator and granulating and drying the resultant preparation, wherein said disintegrator is low-substituted hydroxypropylcellulose having 5 to 16% by weight of hydroxypropoxyl groups, a loose bulk density of 0.40 g/ml or higher and a tapped bulk density of 0.60 g/ml or higher, and wherein the low-substituted hydroxypropylcellulose has a degree of compression of 35% or less, wherein degree of compression (%) is [(tapped bulk density-loose bulk density)/tapped bulk density] x 100.

## Patentansprüche

1. Festes Präparat, umfassend ein schwer lösliches Arzneimittel, ein magensaftresistentes Polymer, einen Hilfsstoff und ein Sprengmittel, wobei ein gemischtes Pulver, das mindestens den Hilfsstoff und das Sprengmittel umfasst, teilweise oder vollständig mit einer festen Dispersion bedeckt ist, die das schwer lösliche Arzneimittel und das magensaftresistente Polymer umfasst, wobei das Sprengmittel niedrig substituierte Hydroxypropylcellulose mit 5 bis 16 Gew.-% Hydroxypropoxyl-Gruppen, einer losen Schüttdichte von 0,40 g/ml oder höher und einer gerüttelten Schüttdichte von 0,60 g/ml oder höher ist, und wobei die niedrig substituierte Hydroxypropylcellulose einen Verdichtungsgrad von 35 % oder weniger aufweist, wobei der Verdichtungsgrad (%) [(gerüttelte Schüttdichte - lose Schüttdichte)/gerüttelte Schüttdichte] x 100 ist.

2. Festes Präparat nach Anspruch 1, wobei ein Gehalt des magensaftresistenten Polymers 1 bis 37 Gew.-% beträgt und ein Gehalt des Sprengmittels 15 bis 50 Gew.-% beträgt.

3. Festes Präparat nach Anspruch 1 oder 2, wobei das magensaftresistente Polymer ausgewählt ist aus der Gruppe bestehend aus Celluloseacetatphthalat, Celluloseacetattrimellitat, Celluloseacetatsuccinat, Methylcellulosephthalat, Hydroxymethylcelluloseethylphthalat, Hydroxypropylmethylcellulosephthalat, Hydroxypropylmethylcelluloseacetatsuccinat, Hydroxypropylmethylacetatmaleat, Hydroxypropylmethyltrimellitat, Carboxymethylethylcellulose, Polyvinylbutyratphthalat, Polyvinylalkoholacetatphthalat, Methacrylsäure-/Ethylacrylat-Copolymer und ein Methylacrylsäure-/Methylmethacrylat-Copolymer.

4. Verfahren zum Herstellen eines festen Präparats, umfassend die Schritte des Sprühens einer magensaftresistenten Polymerlösung, in der ein schwer lösliches Arzneimittel dispergiert oder gelöst wurde, auf einem gemischten Pulver, umfassend einen Hilfsstoff und ein Sprengmittel, und des Granulierens und Trocknens des hergestellten Präparats, wobei das Sprengmittel niedrig substituierte Hydroxypropylcellulose mit 5 bis 16 Gew.-% Hydroxypropoxyl-Gruppen, einer losen Schüttdichte von 0,40 g/ml oder höher und einer gerüttelten Schüttdichte von 0,60 g/ml oder höher ist, und wobei die niedrig substituierte Hydroxypropylcellulose einen Verdichtungsgrad von 35 % oder weniger aufweist, wobei der Verdichtungsgrad (%) [(gerüttelte Schüttdichte - lose Schüttdichte)/gerüttelte Schüttdichte] x 100 ist.

## Revendications

1. Préparation solide comprenant un médicament faiblement soluble, un polymère gastro-résistant, un excipient et un désagrégeant, dans lequel une poudre mélangée comprenant au moins l'excipient et le désagrégeant est partiellement ou entièrement couverte d'une dispersion solide comprenant le médicament faiblement soluble et le polymère gastro-résistant, ledit désagrégeant étant une hydroxypropylcellulose de basse substitution ayant 5 à 16 % en poids de groupes hydroxypropoxyle, une masse volumique apparente non tassée de 0,40 g/ml ou plus et une masse volumique apparente tassée de 0,60 g/ml ou plus, et dans laquelle l'hydroxypropylcellulose de basse substitution a un degré de compression de 35 % ou moins, le degré de compression (%) étant [(masse volumique apparente tassée - masse volumique apparente non tassée)/masse volumique apparente tassée] x 100.

**2.** Préparation solide selon la revendication 1, dans laquelle la proportion dudit polymère gastro-résistant est de 1 à 37 % en poids et la proportion dudit désagrégeant est de 15 à 50 % en poids.

**3.** Préparation solide selon la revendication 1 ou 2, dans laquelle ledit polymère gastro-résistant est sélectionné dans le groupe comprenant : acétophtalate de cellulose, acétotrimellitate de cellulose, acétosuccinate de cellulose, phtalate de méthylcellulose, phtalate de diéthyle d'hydroxyméthylcellulose, phtalate d'hydroxypropylméthylcellulose, acétosuccinate d'hydroxypropylméthylcellulose, acétomaléate d'hydroxypropylméthyle, trimellitate d'hydroxypropylméthyle, carboxyméthyléthylcellulose, butyrate phtalate de polyvinyle, acétophtalate de poly(alcool de vinyle), copolymère d'acide méthacrylique / acrylate d'éthyle, et copolymère d'acide méthacrylique / méthacrylate de méthyle.

**4.** Procédé pour produire une préparation solide, comprenant les étapes consistant à pulvériser une solution de polymère gastro-résistant, dans laquelle un médicament faiblement soluble a été dispersé ou dissous, sur une poudre mélangée comprenant un excipient et un désagrégeant, et granuler et sécher la préparation résultante, ledit désagrégeant étant une hydroxypropylcellulose de basse substitution ayant 5 à 16 % en poids de groupes hydroxypropoxyle, une masse volumique apparente non tassée de 0,40 g/ml ou plus et une masse volumique apparente tassée de 0,60 g/ml ou plus, et l'hydroxypropylcellulose de basse substitution ayant un degré de compression de 35 % ou moins, le degré de compression (%) étant [(masse volumique apparente tassée-masse volumique apparente non tassée)/masse volumique apparente tassée] x 100.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005517690 PCT
- JP 52626421993 B
- JP 2004067606 A
- WO 03063831 A

### Non-patent literature cited in the description

- **HIRASAWA et al.** *Journal of the Pharmaceutical Society of Japan,* 2004, vol. 124 (1), 19-23
- *Journal of the Pharmaceutical Society of Japan,* 2004, vol. 124 (1), 19-23